Europäisches Patentamt

**European Patent Office**

**Office européen des brevets**

(19)

(11) Veröffentlichungsnummer: **0 019 827**
A1

(12) ## EUROPÄISCHE PATENTANMELDUNG

(21) Anmeldenummer: **80102759.0**

(22) Anmeldetag: **19.05.80**

(51) Int. Cl.³: **G 01 R 33/06, A 61 B 5/00**

(30) Priorität: **28.05.79 DE 2921546**

(43) Veröffentlichungstag der Anmeldung: **10.12.80**
**Patentblatt 80/25**

(84) Benannte Vertragsstaaten: **DE FR GB IT**

(71) Anmelder: **SIEMENS AKTIENGESELLSCHAFT Berlin und München, Postfach 22 02 61, D-8000 München 22 (DE)**

(72) Erfinder: **Nickel, Bernd, Dipl.-Ing., Goldregenstrasse 2, D-6143 Lorsch (DE)**

(54) **Anordnung zur Erfassung der Feldstärke eines Felderzeugers mittels eines Hallgenerators.**

(57) Zur Erfassung relativ schwacher Feldstärken wird vorgeschlagen, zur Ansteuerung des Hallgenerators (5) pulsförmige Signale vorzusehen. Bei konstantem Feld können diese eine beliebige Pulsfolgefrequenz haben, bei wechselndem Feld haben sie wenigstens die doppelte Frequenz als die im Nutzsignal vorkommende Frequenz. Das Tastverhältnis der Impulse ist ferner so gewählt, daß der Impulsenergiegehalt eines Impulses gleich oder kleiner ist als die effektive Steuerleistung des Hallgenerators (5). Mit der vorgeschlagenen Anordnung läßt sich die Empfindlichkeit von Hallgeneratoren um ein Vielfaches erhöhen. Die Anordnung kann überall dort, wo sehr schwache Feldstärken zu messen sind, angewendet werden. Eine bevorzugte Anwendung liegt in der Elektrognathographie.

SIEMENS AKTIENGESELLSCHAFT     Unser Zeichen
Berlin und München            VPA 79 P 5044 EUR

Anordnung zur Erfassung der Feldstärke eines Felderzeugers mittels eines Hallgenerators

Die Erfindung bezieht sich auf eine Anordnung zur Erfassung der Feldstärke eines Felderzeugers mittels eines Hallgenerators.

Bei sehr schwachen Feldstärken, z. B. wenn der Felderzeuger vom Meßwertaufnehmer relativ weit entfernt ist oder wenn das maximal ausnutzbare Feld von vorneherein schon sehr schwach ist, reicht häufig die Empfindlichkeit der verwendeten Hallgeneratoren in der bisher üblichen Anordnung nicht aus, um ein zufriedenstellendes, für eine weitere Auswertung brauchbares Nutzsignal zu bekommen.

Aufgabe der Erfindung ist es demnach, eine Anordnung der eingangs genannten Art anzugeben, mit der die Empfindlichkeit von Hallgeneratoren derart verbessert werden kann, daß auch bei sehr schwachen Feldstärken noch auswertbare Nutzsignale zu erzielen sind.

Rp 5 Rl / 23.5.1979

Das gestellte Ziel läßt sich gemäß der Erfindung dadurch erreichen, daß zur Ansteuerung des Hallgenerators
pulsförmige Signale vorgesehen sind, mit einer beliebigen Pulsfolgefrequenz (fi) bei konstantem Feld und
einer zumindest doppelt so hohen Pulsfolgefrequenz als
die im Nutzsignal vorkommende höchste Frequenz einer
Feldstärkeänderung bei wechselndem Feld, und daß das
Tastverhältnis der pulsförmigen Signale so groß ist,
daß der Energiegehalt der Impulse gleich oder kleiner
der (erlaubten) effektiven Steuerleistung des Hallgenerators ist.

Durch die erfindungsgemäß vorgeschlagenen pulsförmigen
Ansteuerimpulse kann der Impulsspitzenstrom wesentlich
höher gewählt werden als der maximal erlaubte Gleichstrom nach bisheriger Anwendungsart. Je extremer das
Tastverhältnis, d. h. je kürzer die Impulsdauer und je
länger die Impulspause ist, um so höher kann die maximale Amplitude des Steuerstromes angesetzt und damit
die Empfindlichkeit des Meßwertaufnehmers gesteigert
werden. Die Impulsform kann an sich beliebig gewählt
werden, vorteilhaft ist es jedoch, eine rechteckförmige Impulsform vorzusehen. Die Abtastfrequenz kann ebenfalls beliebig gewählt werden, im beschriebenen Anwendungsbeispiel ist jedoch eine Frequenz von 50 Hz vorteilhaft. Die Abtastfolge verläuft dann nämlich synchron
zur Netzfrequenz, wodurch sich eventuelle Störsignale,
wie sie beispielsweise von vorhandenen Netzleitungen
oder Transformatoren ausgehen, unterdrücken lassen.

Zur Auswertung der Nutzsignale werden die pulsförmigen
Signale vorteilhafterweise auf eine Spitzenwertgleichrichterschaltung gegeben, die vorzugsweise eine Sample-
&-Hold-Schaltung ist.

0019827

3 –    VPA 79 P 5044 EUR

Ein Ausführungsbeispiel der Erfindung wird nachstehend anhand der Zeichnung näher erläutert, wobei dieses Ausführungsbeispiel auf die technische Anwendung bei einem Elektrognathographiegerät, wie es in der deutschen Patentanmeldung P 28 14 551.9 (VPA 78 P 8905) beschrieben ist, abgestellt ist. Bei solchen Geräten ist die Entfernung zwischen dem Magnet als Felderzeuger und dem Hallgenerator als Feldaufnehmer relativ groß. Das Erdmagnetfeld liegt hier eine Größenordnung höher als das bei einem solchen Gerät vorhandene, vom Magneten ausgehende Nutzfeld.

In der Zeichnung zeigen:

Fig. 1 ein Diagramm zur Erläuterung,

Fig. 2 ein Prinzipschaltbild nach der Erfindung,

Fig. 3 ein Ersatzschaltbild für die Position 7 in Fig. 2.

Geht man zunächst davon aus, daß entsprechend der Formel $U_H = k \cdot I \cdot B$ das zu erhaltende Nutzsignal proportional zur Feldstärke B, dem Steuerstrom I, und den Eigenschaften (k) des Hallgenerators ist, so kann die Empfindlichkeit zunächst durch Erhöhung des Steuerstromes verändert werden. Der maximal mögliche Steuerstrom ist jedoch durch den Hallgenerator selbst begrenzt. Benutzt man anstelle von Gleichstrom zur Ansteuerung pulsförmige Signale wie erfindungsgemäß vorgeschlagen wird, so kann der Impulsspitzenstrom wesentlich höher gewählt werden als der maximal erlaubte Gleichstrom.

- 4 -          VPA 79 P 5044 EUR

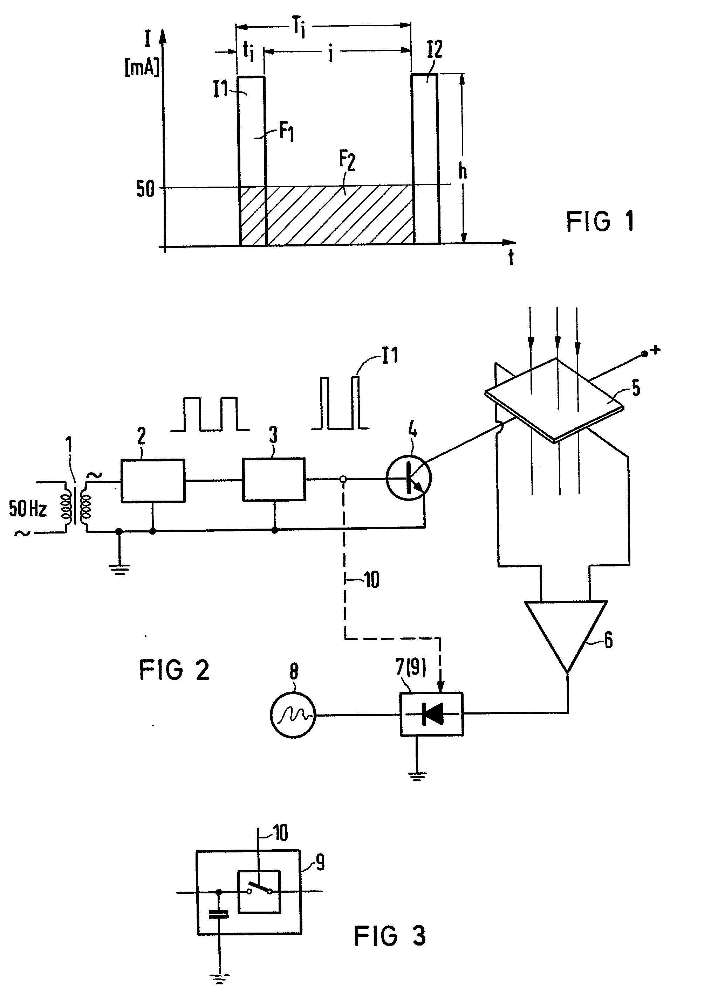

In Fig. 1 der Zeichnung ist dies veranschaulicht. Die Impulse $I_1$, $I_2$ haben im Anwendungsfall eine Impulsbreite $t_i$ von 0,5 ms und eine Impulsperiode $T_i$ von 20 ms. Das Tastverhältnis (Impulsbreite zu Impulsintervall) $t_i/i$ ist dabei so zu wählen, daß die erlaubte effektive Steuerleistung des Hallgenerators nicht überschritten wird, d. h. die mit $F_1$ bezeichnete Fläche darf nicht größer sein als die mit $F_2$ bezeichnete Fläche, die der Steuerleistung des Hallgenerators bei Gleichstrom entspricht. Je extremer das Tastverhältnis, d. h. je kürzer die Impulse und je länger die Impulspause gewählt ist, um so höher kann die maximale Amplitude des Steuerstromes angesetzt und damit die Empfindlichkeit gesteigert werden.

Das Ausgangssignal entspricht in der Form dem Ansteuersignal. Die Impulsfolgefrequenz der Ansteuerimpulse kann bei einem konstanten Feld, z. B. bei einer Messung des Erdmagnetfeldes, beliebig gewählt werden, bei wechselnder Feldstärke ist die Impulsfolgefrequenz jedoch mindestens doppelt so hoch anzusetzen als die größte, im Nutzsignal vorkommende Frequenz. Geht man davon aus, daß bei einem Elektrognathographiegerät, wie es in der genannten deutschen Patentanmeldung P 28 14 551.9 beschrieben ist, die größte Nutzsignalfrequenz bei einer Kaubewegung bei etwa 5 bis 10 Hz liegt, so sollte die Abtastfrequenz höher als 20 Hz liegen. Vorteilhaft ist eine Abtastfrequenz von 50 Hz, weil hier die Abtastfolge synchron zur Netzfrequenz verläuft und sich dadurch eventuelle Störsignale, wie sie etwa durch Netzleitungen oder durch Transformatoren verursacht werden, automatisch unterdrücken lassen.

- 5 -        VPA 79 P 5044  EUR

Die Fig. 2 zeigt anhand eines Blockschaltbildes die zur Verbesserung der Empfindlichkeit vorgeschlagene Ansteuerschaltung. Die von einem Wechselstromtransformator 1 mit der Frequenz von 50 Hz erzeugten Wechselstromsignale werden zur Impulsformung zunächst einem Schmitt-Trigger 2 zugeführt. Eine monostabile Kippstufe 3 liefert dann die Ansteuerimpulse in dem gewünschten Tastverhältnis, die anschließend über einen Transistor 4 dem Hallgenerator 5 zugeführt werden.

Im vorliegenden Ausführungsbeispiel beträgt der Impulsspitzenstrom 250 mA. Im Vergleich zu dem maximal erlaubten Gleichstrom der durch den Hallgenerator, wie er für den vorliegenden Verwendungszweck verwendet wird, fließt, (etwa 25 mA) wird dadurch eine um das zehnfache höhere Meßempfindlichkeit erreicht.

Die durch die genannte Schaltung erzielbaren Impulssignale geben nun eine Information über die Größe bzw. Form (Hüllkurve) des Nutzsignals, welches aus dem Hallgenerator 5 erhalten wird. Um die Impulse in ein Nutzsignal umzuwandeln, werden diese zunächst über einen Verstärker 6 einem Spitzenwertgleichrichter 7 zugeführt. Die hieraus erhältlichen Signale werden sodann in einem geeigneten Meßinstrument 8 angezeigt.

Als optimale Verwendungsform eines Spitzenwertgleichrichters kann eine an sich bekannte Sample-&-Hold-Schaltung, wie sie in Fig. 3 im Prinzip dargestellt und mit der Position 9 bezeichnet ist, benutzt werden. Das in dieser Schaltung enthaltene Schaltelement ist, wie in Fig. 2 gestrichelt eingezeichnet, bei dieser Ausführung über die Leitung 10 von der Ansteuerschaltung aus in deren Takt anzusteuern.

0019827

Das vorgeschlagene Prinzip der Verbesserung der Empfindlichkeit von Hallgeneratoren ist nicht auf das beschriebene Ausführungsbeispiel beschränkt, es kann vielmehr überall dort eingesetzt werden, wo sehr schwache Feldstärken gemessen werden sollen, z. B. auch bei Magnetbändern, Messung des Erdmagnetfeldes, oder dergleichen.

<u>Patentansprüche</u>

1. Anordnung zur Erfassung von einem Felderzeuger ausgehender Feldstärken mittels eines Hallgenerators, d a d u r c h  g e k e n n z e i c h n e t ,  daß zur Ansteuerung des Hallgenerators (5) pulsförmige Signale ($I_1$, $I_2$) vorgesehen sind, mit einer beliebigen Pulsfolgefrequenz (fi) bei konstantem Feld und einer zumindest doppelt so hohen Pulsfolgefrequenz als die im Nutzsignal vorkommende höchste Frequenz einer Feldstärkeänderung bei wechselndem Feld, und daß das Tastverhältnis ($V_T$) der Impulse ($I_1$, $I_2$) so gewählt ist, daß der Impulsenergiegehalt ($F_1$) eines Impulses ($I_1$) gleich oder kleiner ist als die effektive Steuerleistung ($F_2$) des Hallgenerators (5).

2. Anordnung nach Anspruch 1, d a d u r c h  g e - k e n n z e i c h n e t ,  daß als Ansteuerimpulse Rechteckimpulse ($I_1$, $I_2$) vorgesehen sind.

3. Anordnung nach Anspruch 1 oder 2, d a d u r c h  g e k e n n z e i c h n e t ,  daß die Pulsfolgefrequenz (fi) 50 Hz beträgt.

4. Anordnung nach einem der Ansprüche 1 bis 3, d a - d u r c h  g e k e n n z e i c h n e t ,  daß die am Hallgenerator (5) erhaltenen Impulse mittels eines Spitzenwertgleichrichters (7) in auswertbare Nutzsignale, z. B. in eine Hüllkurve, umgewandelt werden.

5. Anordnung nach Anspruch 4, d a d u r c h  g e - k e n n z e i c h n e t ,  daß als Spitzenwertgleichrichter eine Sample-&-Hold-Schaltung (9) vorgesehen ist, die durch die Ansteuerimpulse ($I_1$, $I_2$) synchron zur Impulsfolgefrequenz (fi) des Nutzsignals des Hallgenerators (5) gesteuert wird.

FIG 1

FIG 2

FIG 3

## EINSCHLÄGIGE DOKUMENTE

| Kategorie | Kennzeichnung des Dokuments mit Angabe, soweit erforderlich, der maßgeblichen Teile | betrifft Anspruch |
|---|---|---|
| X | <u>AT - B - 299 375</u> (SIEMENS)<br>* Ganze Druckschrift *<br>& DE-A1-1 919 440<br>-- | 1 |
| D,P | <u>DE - A1 - 2 814 551</u> (SIEMENS)<br>(18-10-1979)<br>* Ganze Druckschrift *<br>---- | 1-5 |

**KLASSIFIKATION DER ANMELDUNG (Int.Cl. 3)**

G 01 R 33/06
A 61 B 5/00

**RECHERCHIERTE SACHGEBIETE (Int. Cl. 3)**

G 01 R 31/00
G 01 D 5/00
A 61 B 5/00

**KATEGORIE DER GENANNTEN DOKUMENTE**

X: von besonderer Bedeutung

A: technologischer Hintergrund

O: nichtschriftliche Offenbarung

P: Zwischenliteratur

T: der Erfindung zugrunde liegende Theorien oder Grundsätze

E: kollidierende Anmeldung

D: in der Anmeldung angeführtes Dokument

L: aus andern Gründen angeführtes Dokument

&: Mitglied der gleichen Patentfamilie, übereinstimmendes Dokument

X   Der vorliegende Recherchenbericht wurde für alle Patentansprüche erstellt.

| Recherchenort | Abschlußdatum der Recherche | Prüfer |
|---|---|---|
| WIEN | 28-08-1980 | KUNZE |

EPA form 1503.1   06.78